# EUROPEAN PATENT APPLICATION

(11) **EP 1 659 407 A1**
(43) Date of publication of application: **24.05.2006**
(21) Application number: 05257128.8
(22) Date of filing: 18.11.2005
(51) Int. Cl.: G01N 33/96, G01N 33/543, G01N 33/558

(54) **Diagnostic control system**

(30) Priority: 19.11.2004 US 628962 P
(71) Applicant: Norwegian Antibodies AS, 1430 As (NO)
(72) Inventor: Øistein, Larsen, N-1430 ÅS (NO)
(74) Representative: Baldock, Sharon Claire

(57) **Abstract**

A novel control system applicable in immunochromatographic analysis is provided, wherein the control system comprises a binding pair consisting of an antigen and an antibody, and wherein at the antigen a species-specific substance, and further provided that the antigen is not an antibody per se.

## Description

The present invention relates to a control system applicable for in vitro immunochromatographic analysis, comprising a binding pair constituted by an antigen and antibody towards said antigen, and wherein said antigen origins from a species different from the species from which the analyte origins, and provided that said antigen is not an antibody per se.

Furthermore, in another aspect, the present invention relates to a control system for immunochromatographic systems, comprising a binding pair, where one of the members of the said binding pair is immobilized on a coloured microparticle, and wherein the said coloured microparticle is of a different colour compared with the colour of the microparticle which in said immunochromatographic system is used to visualize the analyte to be detected by said immunochromatographic system.

### Bacground of the invention.

Immunochromatografic techniques have been used for decades as the main prinsiple in many in vitro diagnostic test systems. In general, these test systems are based on the utilisation of specific compounds (binder) attached to a solid support which recogninse and bind to specific analyte(s) to be detected. The formed analyte-binder complex is then visualized using various methods. Such methods and devices also disclose various control means to demonstrate proper functioning of the test system. The main priciples of such immunochromatographic systems and devices are e.g. disclosed in US 4855240, US 4855210, US 4703017, US 4376110, US 6537828, EP 291194 or EP A 1 810 436.

Thus, various systems based on immunochromatograpy are described in the prior art. Starting with two out of the world's three largest diagnostics firms and their lists of products, we see that this form of qualitative thin-layer immunochromatography is used extensively. Bayer, USA, markets Clinitek hCG urine test and Clinitek Microalbumin urine test. Abbott Laboratories USA markets Fact Plus Pregnancy Test, TestPack hCG Combo, TestPack Chlamydia, TestPack Strep A, TestPack Rotavirus, and TestPack RSV. Of the smaller but more specialized firms we could mention Nubenco Medical International, USA, which markets these types of tests for hCG, LH, Chagas, Chlamydia, Cholera, CK MB, Dengue, Myoglobin, Strep A, Hepatitis B antigen, Tropnin I, Hemoglobin in stool, as well as for antibodies for Dengue, Helicobacter pylori, Hepatitis B antibodies, mononucleosis antibodies, antibodies for Treponoma pallidum and Mycobacteria tuberculosis, and also for detection of the tumor markers Alpha Fetoprotein, Carcinoembryonal antigen and Prostata spesific antigen. Millipore Inc., USA, which is a specialized producer of filter materials, markets full hardware «assembly kits» for these types of tests from their OEM-department, so-called HiFlow assembly kits. Pall Gelman plc, UK, has even issued a manual for manufacturing such products, viz. their brochure «Immunochromatographic, Lateral Flow or Test Strip Development Ideas» which can also be downloaded from their Internet site. Acon Laboratories, Inc., USA, has its own large-scale production and sales of these types of tests, especially to the Chinese market, but also deliver tests, so-called OEM, to other firms for resale under the customer's trademark.

Apparatuses for measuring the intensity of these stripes or patterns have also been constructed, but it has been difficult to design chemicals and devices providing sufficiently precise and accurate results. Highly sophisticated technologies have been developed to overcome these limitations, viz. typically US 6136610: «Method and apparatus for performing a lateral flow assay» by Polito & al. It is evident from US 6136610 that more complicated and advanced methods and apparatuses are needed to make this method quantitative, and it is very complicated and costly to achieve industrially reproducible precision and accuracy, until now it actually has not been feasible in a commercial context.

Roche Diagnostics has developed a variety of this chromatographically principle in which the intensity of coloration in the testing section to which the signal-providing substances proceed is used as a semi-quantitative measure for determining albumin in urine. Diabetes care, vol. 20, number 11, pp. 1642 - 1646, describes this.

Common for most of the diagnostic test based on immunochromatography is the need of a control test to ensure that the test is working. Such control system is also necessary to prevent the risk of a false negative result etc. Therefore, the immunochromatographic test usually comprises a control reagent and a detector reagent, wherein the control reagent is immobilized in a control region adjacent to the detecting region of the test strip wherein the analyte binding substance is present.

The chromatographic analysis commonly utilizes antibodies as control reagents. For example, in vitro solid phase assays, such as the diagnostics test strips known in the prior art, comprise immobilized antibodies e.g. anti mouse IgG on the control line as well as antibodies immobilized on the test line. The anti-mouse IgG is then visualized by the binding of an antibody as a detector reagent. In some systems, the detector reagent, i.e. the antibody, and the analyte detector reagent is the same, for example a colloidal anti-analyte antibody.

However, the use of e.g. anti-IgG's on the control line causes various disadvantages since antibodies present in the test sample may bind to and block the binding of the labeled antibodies present in the test system. On the other hand, if the test sample contain saturating amounts of analyte (i.e. the compound to be detected), this may cause all the labeled anti-analyte antibodies present in the test to bind to the analyte. Furthermore, it cause all the antibodies present in the test bind to the reagent in the test line, leaving no labeled antibodies left for binding to the anti-antibodies in the control line. A signal on the control line is therefore hindered, making the result unreliable.

Protein A is a surface protein produced by *Staphylococcus aureus*. The fact that Protein A binds to the Fc portions of immunoglobulins, specifically IgG from various species, has rendered it possible to utilize this protein as a mean for the purification and recovery of both polyclonal and monoclonal antibodies.

Further, anti-Protein A-antibodies have been used as a control system in various solid phase assays; Ampac provides a lateral flow chromatoghrafic immunoassay for the detection of specific anti-HCV-antibodies. The controll line of this test comprises immobilezed rabbit anti-proein A antibodies. The object of this control line is that protein A-colloidal gold conjugates contained in a conjugate pad will bind to the anti-protein A antibodies and thus indicate that the test is correctly performed (cf. http://www.ampacasiapacific.com/).

CFRD also provides a diagnostic immunochromatographic based test for Hepatitis C antibodies in serum and hole blood (cf. http://www.cfrd.org/en/hepatit_C.html). Also in this test, anti-Protein A antibodies are used as the control reagent which is immobilized onto the control line, and wherein Protein A is used as the detector reagent.

The use of anti-Protein A antibodies immobilized on the control line is further utilized in the chromatographic immunoassay for the detection of antibodies to Human Immunodeficiency Virus type-1 and -2 provided by West Wind Plus, Inc (cf. http://www.eazy-sure.com/one-step/infectious/hivr1-2.htm).

However, the utilization of anti-Protein A in the control line causes major disadvantages. Because of the capability of Protein A to bind to the Fc-region of various antibodies, the labeled Protein A in the test may bind to antibodies in the test sample. If the test sample comprise saturating amounts of antibodies, all colloidal Protein A may be bound to antibodies in the test sample thus preventing the production of a signal on the control line. This makes such test unrealible. Also, if anti-analyte antibodies are immobilized on the test line, e.g. anti HCG-antibodies, the colloidal protein A may bind to the Fc region of the testline antibodies and thus cause the production of false positive results.
The utilizing of protein A as a control detector in a control system of chromathografic analysis is therefore not at all funcitonal.

US 5541059 discloses an immunoassay device having an internal controll of protein A, wherein protein A is immobilized in an control region of the test strip, e.g using protein A as the control reagent. However, contrary to the present invention, the system disclosed in US 5547059 utilizes human antibodies precent in the test sample in the detection and visualization of the control line. Thus, the control system disclosed in US 5547059 expliot the fact that Protein A is able to bind to the Fc part of antibodies in general. Therefor, the visualization of the control region according to the system disclosed in said patent depends on the presence of antibodies in the test sample. The dependence of the presence of antibodies in the test sample renders the system disclosed in US 5547059 less suitable due to the possiblilities of false negativ results of the controll line. If the test sample comprises an excess of antibodies for some reason ( e.g. being a blood sample oer urin from a person with heavy proteinuria) a blocking of the Protein A binding occurs, and the signal from the control line may not develop. The control system disclosed in US 5547059 is also a more complicated and expensive system.

US 5,766,961, US 5,770,460 and EP 0 566 695 disclose chromatografic immunoassays comprising independent control line systems using avidin and streptavidin in combination with biotinylated compounds to obtain an independent control line. However, the introduction of biotinylated substances, avidin and straptavidin, and the need of purification of such substances render the production of such systems highly complex and very uneconomical.

Avidin is an egg white protein which previously has been suggested as a control reagent in immunochromatograpic analysis. In US 6537828, the immobilization of avidin on immunochromatographic devices as a control reagent is disclosed. The visualization of the control line depends on the binding of biotinylated colloid particles present in the test reagents which binds to the avidin. This is however not a sufficiently cost efficient system.

Until now, all control lines have had the same colour as the test lines in lateral flow tests. E.g. both lines have been red or both lines have been blue. Since these tests often is performed by people not having special skill in the area, and maybe have not done any such lateral flow test before, it has been a challenge to teach the perforemers of the test the interpretation of the test line and the control line. If two lines are visible after the performance of the test method, the result shall be interpretated as a demonstration of presence of the anlyte substance in the test sample and also a demonstration of a correct performance of the test. However, if only one line is present, it usually means that there is no analyte substance (or less than the threshold value) in the test sample, but the test has been correcly performed. If - however - the control line does not appear, the visible test line can mistakenly be understood as being the control line, leading to a misreading of the test result. It has been difficult to circumvent this problem, since all tests have had identical colours from the test line particles and the control line particles. Some test producers have tried different shapes on the control line and the test line to overcome this problem. For example, various shapes of the control line and test line is disclosed in US 5,541,059, cf. figure 1 - 7. However, suchs approach requires a somewhat more complex production line for the making of the control and the test areas is necessary and is thus less favourable.

There is clearly a need for better and more reliable control systems for chromatographic immunoanalysis.

### Summary of the invention

In view of the above, it is an object of the present invention to provide reliable control systems for analytical immunochromatographic systems. This is accomplished by the utilization of a binding pair as the main control components, wherein the binding pair constitutes of an antigen being of different origin than the analyte/test sample, and an antibody, provided that the antigen is not an antibody per se. In addition, a major advantage of the present invention is that present control system renders the visualization of a control region in an immunochromatographic analysis independent of antibodies or other components naturally present in a test sample.

The present invention therefore provides a control system for immunochromatographic analysis, wherein the reliability of the test is ensured by utilizing a control reagent and control detector constituting the binding pair, and wherein the binding pair comprises of an antigen and an antibody toward said antigen, wherein said antigen is a specie specific antigen of different origin than the analyte, and provided that the antigen is not an antibody per se.

In one aspect of the present invention, the control reagent is an immobilized antibody, which is visualized upon binding to a control detector then being an antigen having a different origin than the test analyte/test sample.

In another aspect, the control reagent is an antigen, which is visualized upon binding to a detector reagent being an antibody. Thus, the control system according to the present invention is independent of whether the antigen is used as the control reagent or as the control detector.

Furthermore, a chromatographic control system is provided, wherein the control region and the test line, upon visualization form different coloured signals on the control region when detected by the control reagent compared with the coloured signals formed upon the binding of the analyte detector to the analyte on the test line. By utilizing different labels attached to the detector reagent and the analyte detector, the control system according to the present invention is applicable for chromatographic test devices having a control line signal forming a different colour than the test line signal. More specifically, the present invention provides a control system wherein one of the members of the binding pair is immobilized on a coloured microparticle, and wherein said coloured microparticle has a colour which is different from the colour of the microparticle which in said immunochromatographic system is used to visualize the analyte to be detected by said immunochromatogarphic system.

Furthermore, the present invention also provides a novel solid phase immunochromatographic device comprising the control system of the present invention.

Hemocyanin and protein A are proteins which are very antigenic, thus being very efficient in inducing antibodies after immunization in the animal producing the antibodies to be used in this invention. Furthermore, they are inexpensive, and therefore efficient on the use forlarge scale antigen affinity chromatography of the antibodies. In the use of this invention, it has been demonstrated that the use of antigen affinity chromatography purifies antibodies should be used to obtain good results. Furthermore, there is a need for low cost reagents in this competetive market, so more expensive proteins are prohibited. They cannot be used neither for striping on the nitrocellulose, nor the immobilisation on the dyed particles nor in the large scale antigen affinitychromatography purification processes needed, without bringing the over all reagent costs up to a too high level.

The present inventionis thus a surprising efficient result obtained when hemocyanina or Protein A is used. Protein A or hemocyanin and the antobides reactive with these proteins respectively providevery efficient binding pairs for a very robust formation of control lines. The use of protein A or hemocyanin provides very affordable high quality antigen affinity prurified anibodies, very efficient and affordable reagents for striping of control lines on lateral flow immunochromatography tests, very efficient and afforable dyed control particles. The combined result is a surprisingly overall very robust, independant and affordable control line system for industrial production of control line systems for lateral flow immunochromatography systems.

This system have superior advantages over the prior art test systems. The present control system prevents the production of false positive results and situations were no signal incorrectly is produced on the control line which may occur with the test described in the prior art.

### Brief description of the figures

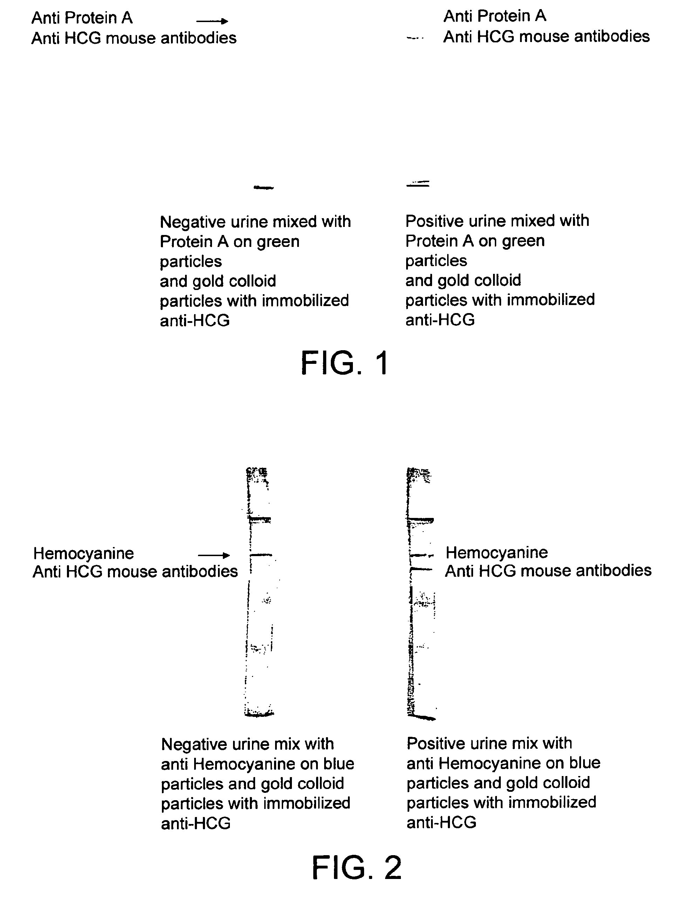

### Figure 1 demonstrates the use of anti Protein A antibodies on the control line and Protein A immobilized on green latex particles.

### Figur 2 demonstrates the use of anti Hemocyanin antibodies on the control line and Hemocyanin immobilized on green latex particles.

### Detailed description of the invention.

The present invention provides control systems for immunochromatographic analysis and devices, wherein the reliability is ensured by utilizing a binding pair, constituted by 1) an antigen of different origin than the analyte to be detected, provided that said antigen is not an antibody per se, and 2) an antibody towards said antigen.

The immunochromatographic control system of the present invention is applicable in various common immunochromatic analysis systems, e.g like various known forms of qualitative thin-layer immunochromatographic devices.

The commonly market chromatographic test systems comprise a sample applying region. Upon application of a sample suspected to comprise an analyte, the test sample solution (e.g. blood sample or urine) moves in the direction of a detection region due to capillary flow. The detection region commonly comprises an analyte binding substance, which upon binding the analyte is recognized and visualized by a second labeled analyte detector reagent. In addition, such test systems usually comprise a control region wherein an immobilized control reagent is applied. The detection and visualization of the control reagent may be accomplished by the binding of at least one labeled control reagent binding substance. In common control systems, the detection of the control substance is often dependent of the presence of substances present in the test sample, such as antigens or antibodies. The visualization is then accomplished by the binding of a second control labeled control detector. In other known systems, the control reagent is an antibody being detected by another labeled antibody (secondary antibody) present in the test reagents. The object of the visualization of a control region/control line is to demonstrate that the test is functional and correctly performed.

According to the present invention, the control reagent is visualized by the subsequent direct binding of a labeled control detector to the control reagent. Thus, the visualization of the control reagent according to the present invention is independent of any other substances present in the test sample and is less vulnerable to false signals due to e.g. the interference of substances present in the analyte sample.

The present invention provides a novel control system, wherein on of the component of the binding pair is a species-specific antigen being of different origin than the test analyte, and wherein said antigen is not an antibody per se.

The terms "control component" or "component of the control system", as used herein, represents a control reagent and a control detector of an immunochromatographic analysis as defined below. The control reagent and the control detector constitute the binding pair of the present control system.

The terms "antigen" as used herein represents any compound, which is derived from another species than the test sample/analyte to be analyzed and which is not an antibody per se. The antigen is thus a species-specific substance, e.g. a species-specific protein. The "antigen" can be derived from various organisms, e.g. from an organism belonging to the taxonomic group of protostomia, such as belonging to the taxonomic group phylum molluscan, or from the various taxonomic group of deuterostomia, such as belonging to the group chordate or the subphylum vertebrata such as from aves, reptila or amphibian. The "antigen" may also be derived from other taxonomic groups such as plantae or fungi. Non-limiting examples of "antigen" is for example proteins and glycoproteins, lipids, peptides, nucleic acids and derivatives of proteins, glycoproteins, lipids, peptides and nucleic acids.

According to a preferred embodiment of the present inventions, the antigen is selected from the group of the *Staphylococcus aureus* proteins Protein A or protein G, or a hemocyanin, preferably molluscan hemocyanin. Other non-limiting examples of suitable antigens according to the present control system are avidin, optionally deglycosylated, or straptavidin.

Based on the present specification and description of the invention, the person skilled in the art will acknowledge that other substances than those specifically mentioned herein may be useful as an antigen in the control system of the present invention.

Furthermore, the "antigen" may be derived from its natural source or it might be manufactured by utilizing processes well known in the art. For example, in case the antigen is a protein, such as a bacterial protein, e.g. Protein A, the protein may be isolated and further purified from a cell culture, e.g. a culture of *Staphylococcus aureus*. The antigen may also be manufactured by recombinant technology, e.g. by the expression of a Protein A encoding gene sequence in an appropriate expression system according to methods well known to the skilled person. If the antigen is a protein it may also be manufactured by well know peptide synthesis methods.

The term "control reagent" as used herein represents a compound, being one of the binding pair, which is immobilized in the control region/control line of an immunochromatographic analysis device. The control reagent may be an antigen as defined above which is recognized and visualized by a detector reagent, e.g. a labeled antibody. Conversely, the control reagent may also be an antibody, immobilized on the control region/control line, which binds to the control detector, being the antigen.

The control reagent may be immobilized on the control region of an immunoanalysis device by e.g. physical adsorption or by chemical coupling according to methods well known in the art.

The term "control detector" as used herein includes any compound, which recognize and binds to the control reagent in a matter that enables visualization of the formed control reagent - control detector complex, i.e. the antibody-antigen complex. The visualization is a result of labeling of the control detector.

Furthermore, the "control detector" may according to one embodiment of the invention be the antigen as defined above. According to another embodiment of the invention, the control detector may also constitute of an antibody binding to an immobilized antigen (i.e. the control reagent).

Thus as for the control reagent, the control detector may be an antigen or an antibody provided that it is the opposite of the control reagent. That is, if the control reagent is a species-specific antigen, then the control detector may be a labeled antibody recognizing said control reagent. On the contrary, if the control detector is a species-specific antigen, then the control reagent is a labeled antibody.

Thus, the control system according to the present invention is independent of whether said antigen is applied to the control region and then recognized and visualized upon binding of a control detector, or whether the control reagent is an antibody being recognized and visualized by the subsequent binding of the antigen constituting the control detector. In addition, the visualization of the control reagent is independent of the presence of compounds, e.g. antibodies, present in the test sample.

The term "test sample", as used herein, refers to a material suspected of containing the analyte of interest. The test sample may be used directly as obtained from the source or following a preparation process to modify the character of the sample. Such processing methods may inter alia involve filtration, distillation, extraction, and concentration, inactivation of interfering components, the addition of reagents, etc.

The test sample may be derived from any biological source, such as a physiological fluid, such as, for example, blood, saliva, ocular lens fluid, cerebral spinal fluid, sweat, urine; milk, ascites fluid, synovial fluid, peritoneal fluid, amniotic fluid, faeces etc.

Furthermore, other liquid samples besides physiological fluids may be used, such as water, food products, and the like, for the performance of environmental or food production assays.

The analyte to be detected may be any substance of interest such as hormones, various metabolites, antibodies directed to compounds of pathogenic origin such as virus antigens or anti-virus-antibodies, contaminant, etc.

According to the present control system, the control reagent or the control detector can be antibodies recognizing the antigen immobilized on the control region/control line. The term "antibody" or "antibodies" as used herein represents polyclonal and monoclonal antibodies or fragments thereof such as a Fab fragment, and also chimeric antibodies or single chain antibodies. According to a preferred embodiment of the present invention, the antibody is a non-mammalian antibody, preferably an avian derived antibody. The advantageous of using antibodies of avian origin, e.g. chicken antibodies, is among other reasons due to the highly cost-effective production of antibodies from laying hens compared to the traditional production of antibodies in mammalian e.g. by purifying antibodies from blood. When the analyte of interest is of mammalian origin, it is further an advantage that the antibody is derived from a non-mammalian organism.

The detector reagent, independent of being the substance of origin or being a compound (e.g. antibody) recognizing said substance, may be labeled to provide visualizations of the control region/control line.

As used herein, the expression "label" refers to any substance, which is attached to the control detector to render the control reagent-detector reagent complex detectable. The label is thus capable of producing a signal that is detectable by visual or instrumental means. Various labels suitable for use in the present invention are know and include labels which produce signals through either chemical or physical means. Such labels can include enzymes and substrates; chromogens; catalysts; fluorescent compounds; chemiluminescent compounds; radioactive labels; direct visual labels including colloidal metallic particles such as gold colloidal particles, colloidal non-metallic particles such as selenium, dyed or coloured particles such as coloured or colourable organic polymer latex particles, and liposomes or other vesicles containing directly visible substances; etc.

The selection of a particular label is not critical to the present invention, but the label is preferably capable of generating a detectable signal either by itself, such as a visually detectable coloured organic polymer latex particle.

Furthermore, a "labelled antigen" or a "labelled antibody" as used herein means that the antigen or the antibody is bound to or immobilized on a label as defined above. Preferably, the antigen or antibody is immobilized on microparticles such as metallic particle, such as colloidal gold particles, or coloured polymer latex particle, preferably blue or green latex particles.

The label responsible for visualizing the presence of an analyte in the test sample may include a label as defined above, which is identical or different from the label used to detect the control reagent.

According to a preferred embodiment of the present invention, the label responsible for visualizing the presence of an analyte in the test sample is different form the label used to detect the control reagent by means of forming different coloured regions. More preferably, the analyte detector is immobilized on microparticles, which upon binding to an analyte provides a coloured test line, which is distinguishable, compared with the colour formed on the control region upon binding of the control detector to the control reagent. Even more preferably, the control detector is immobilized on green latex particles and the analyte detector is immobilized on blue latex particles or vice versa. The presence of different colours at the control region/control line and the test region/test line of an immunochromatographic device provides for an easy and reliable reading of the results.

According to another embodiment of the invention, the antigen according to the present invention is Protein A of *Staphylococcus aureus,* wherein Protein A is used as the control reagent and wherein labeled anti-protein A-antibodies is used as detector reagent. It is to be understood that the term "Protein A" as used herein includes both naturally occurring Protein A isolated from *Staphylococcus aureus* as well as Protein A or fragments/ analogous thereof produced by recombinant techniques or chemical synthesis. Fragments or analogs of Protein A may be fragments where one or more amino acids have been deleted, substituted or added.

Anti-Protein A-antibodies may be monoclonal or polyclonal or a fragment thereof as defined above and produced by methods well known to the person skilled in the art. Said antibodies may be of mammalian or avian origin. Preferably, the antibodies are of avian origin, e.g. chicken antibodies due inter alia to the highly cost-effective production of antibodies from laying hens compared to the traditional production of antibodies in mammalian e.g. by purifying antibodies from blood. The difference between avian and mammalian antibodies also makes the immunochromatographic analysis more sensitive and provides less background. Furthermore, Protein A is unable to bind to the Fc-portion of a chicken IgY. Methods for the production of antibodies in hen egg are well known to the person skilled in the art. The anti-Protein A-antibodies may be labeled with a detector agent, which upon binding to Protein A produces signals, useful for the visualization of the control line. Preferably, the anti-protein A-antibody is immobilized on microparticles, e.g. gold colloid particles or coloured latex particles.

Another protein derived from *Staphylococcus aureus*, the Protein G may also be used as the antigen of the binding pair according to the present control system. Protein G may be provided by methods well known in the art, e.g. by extraction from a *Staphylococcus* culture or by means of recombinant technology. As for the Protein A, Protein G is preferably used as a control reagent immobilized on a test region of a immunoanalysis strip. The protein G is visualized by anit-protein G-antibodies, preferably anti-Protein G-antibodies of avian origin. Said anti-protein G-antibodies are preferably immobilized on microparticles, more preferrably on gold colloid particles or coloured latex particles.

Both Protein A and Protein G are commersial available and may be purcased from e.g. Sigma, US (http://www.sigmaaldrich.com/).

Hemocyanins are respiratory proteins that use copper binding sites to bind and transport oxygen in blood in a variety of arthropods and molluscs. They typically have high molecular weights and multiple subunits. Hemocyanin from Megathura crenulata, more particularly the keyhole limpet hemocyanin (KLH) is well known and widely used as e.g. a vaccine carrier resulting in an improved response to vaccines. Hemocyanins are commercial available and may be purchased from e.g.

Sigma Inc., US. (http://www.sigmaaldrich.com/). Thus, according to another preferred embodiment of the present invention, the antigen used in the present control system is hemocyanin, more preferably molluscan hemocyanin. In that case, the other control component of the system is an anti-hemocyanin-antibody.

Hemocyanin may be used as the control reagent immobilized on an immunochromatographic test strip or as the labelled control detector according to the present invention. In case the hemocyanin is used as a control detector binding to the anti-hemocyanin-antibodies immobilized on the test region/test line, the hemocyanin is preferably labelled with coloured latex particles.

As for the anti-Protein A-antibody, anti-hemocyanin-antibodies may be monoclonal or polyclonal or a fragment thereof as defined above, and produced by methods well known to the person skilled in the art. Said antibodies may be of mammalian or avian origin, preferably of avian origin, e.g. chicken antibodies. The anti-hemocyanin-antibodies may be labeled whenever said antibodies is used as detector reagents, preferably by immobilization to microparticles, e.g. gold colloid particles or coloured latex particles.

According to another aspect of the invention, the present invention provides a solid phase immunochromatographic test device, wherein the device comprises a control system according to the present invention. The test device according to the present invention is preferably a solid phase chromatographic device, e.g. thin-layer immunochromatographic device.

The solid phase chromatographic devise according to the present invention may comprise an application zone where the test sample, e.g. urine or blood is applied. The application zone often constitutes a sample pad that inter alia facilitates an even and controlled distribution of the test sample further on to a conjugate pad. When the test samples according to one embodiment of the invention enters the conjugate pad, the detector reagent, i.e. the labeled anti-Protein A antibodies, and optionally reagents that recognize and binds to the specific analyte to be detected are solubilized. The sample then moves through a membrane comprising a test line with immobilized analyte binding substances and a distinct control line comprising immobilized Protein A.

Finally, the device according to the present invention may comprise an absorbent pad at the distal end of the device.

Various common materials are suitable for the production and adaptation of sample pads, conjugate pads, adsorbent pads and membranes and thus well known to the person skilled in the art. A full teaching of the subject is found in the Millipore publication Lateral Flow Test Strips, Considerations for Product Development", Lit. No. TB500EN00. Furthermore, Swedish patent application SE20020001738 and 20020607 describes the use of glass materials for such use.

### EXAMPLES

The present invention will now be illustrated by way of examples. Although the examples represent preferred embodiment of the invention (best mode), they are not to be contemplated as restrictive or limiting to the scope of the invention.

### Example 1 Blue latex with immobilised anti- Protein A chicken antibodies.

IgY from egg from hens having been immunised with Protein A in a suspension with Freund's complete adjuvance was isolated by conventional ammonium chloride precipitation technique (and optionally affinity purified by the use of Protein A immobilised in a gel column), all well described in the literature well known to the skilled man of the art. 30 mg of the antibodies were dialysed against 10 mM borate buffer 15 mM NaCl pH = 9.3 for 24 hours, and the volume was adjusted to 15 ml in the same buffer.

Estapor Carboxylated microspheres PSI 90-21 of mean diameter 0.117, 164 equivalents carboxyl moieties per gram latex particles, was purchased from Merck. 90 mg of the particles was dialysed against water for 12 hours. The volume was adjusted to 4.5 ml by adding water.

5 mg 1-ethyl-3(3-dimethylaminopropyl)carbodiimide (abbreviated EDC) was dissolved in 3 ml water at 2 degrees Celsius, and admixed to the particles suspension under agitation. Immediately thereafter, the above-mentioned antibody solution was added under agitation.

After agitation over night, the particle suspension was washed twice by centrifugation in 10 mM borate buffer pH = 9.3 with 15 mM NaCl, 0.09 % Na Azide, 0.1 % Tween 20 and 2 mg/ml chicken albumin, and thereafter twice with the same buffer but without Tween 20 and at pH = 8.5

### Example 2 Gold colloids with immobilised anti- Protein A chicken antibodies.

10 ml of 1 % gold chloride in distilled water was mixed with 1 litre of boiling distilled water. 10 ml of 34 mM sodium citrate, pH adjusted to 4.2 was added and mixed for 20 minutes. Colloidal gold was formed. The suspension was allowed to cool to room temperature. 1 ml of 1 % PEG 20.000 was added, and pH was adjusted to pH = 7.2. The size of the gold colloid particles was measured using the conventional technique by measurement of the ratio of optical density of 540 nm and 600 nm. The method may be adjusted to obtain mean particle size between 30 and 50 nm. Glassware to be used must be siliconized.

The gold colloid particles are labelled with the chicken anti- Protein A antibodies described above, using the method described by Slot and Geuze in Eur. J. Cell. Biol. 38: 87-93, 1985, to the saturation point according the same method. The labelled gold colloids were then suspended in a 10 mM HEPES buffer solution at pH = 7.1, comprising 0.3 M mannitol, 0.05 % PEG 20000 and 0.1 % egg albumin.

Other gold colloids were made by the same method, comprising monoclonal mouse anti- HCG instead of anti-Protein A antibodies.

### Example 3 Immobilising Protein A on the control line.

HighFlow 120 nitrocellulose from Millipore, US, was cut in pieces approximately 4 mm x 70 mm, and an cellulose absorbance pad to absorb was attached to one end (hereafter named and B, the opposite end named end A).

To 10 mM ammonium acetate in water was added 2 vol. % ethanol. To this solution bacterial Protein A from Sigma was dissolved 2 mg per ml. Using a syringe tip, this solution was applied across the nitrocellulose strip, 7 ul per cm, and left to dry for 2 hours at 37 degrees Celsius. On some of the sheets, a test line with anti- human chorion gonadotropin mouse monoclonal antibodies was applied using the same method, the test line being located a few mm upstream from the control line.

Thereafter, end A of the sheets were dipped into a blocking solution comprising 25 mM TRIS buffer, 150 mM NaCl, egg albumin 2 mg/ml, 100 ug/ml of irrelevant IgY, and this blocking solution migrated through the sheet and into the absorbance pad. Thereafter, the sheets were left to dry in 37 degrees Celsius.

Optionally, the blocking solution comprised 5 vol. % of goat blood serum, which gave an excellent blocking. The goat antibodies in the serum did not block the independent Protein A control line. Neither did mammalian antibodies from the test sample (see below). The reason for this is that the irrelevant mammalian antibodies bind to Protein A with their constant chain (Fc chain) at a specific binding site on Protein A, while the antibodies raised by immunisation of the animal with Protein A binds on other epitopes/binding sites of Protein A (whether these are polyclonal or monoclonal of origin). Especially preferred are avian antibodies, since their Fc-chain does not bind to Protein A, and any kind of interfering reactions are thus avoided.

### Example 4 Demonstration of the use of immobilised Protein A on the control line and chicken Anti-Protein A immobilised on coloured latex and gold colloids.

50 ul Urine samples, some comprising 1000 IU/l human chorion gonadotropic hormone (HCG), some without HCG, was mixed with 200 ul of a buffer comprising 25 mM TRIS buffer, 150 mM NaCl, egg albumin 2 mg/ml, 50 ug/ml of irrelevant IgY.
To create a control line, 25 ul of particle suspension comprising either gold colloids or blue latex (see above) with immobilized anti-Protein A antibodies (see above) was mixed with the urine/buffer mixture.

The nitrocellulose sheets, treated as described above, was inserted into the resulting mixture, and the mixture migrated through the sheet, passed the test line (if applied) and the control line with the immobilized protein A, and into the absorption pad. A control line developed. Coloured particles did not bind to the test line if present.
In separate experiments, 25 ul of gold colloid particles with immobilized anti-HCG antibodies was mixed to the said mixture comprising gold colloid particles with immobilized anti- Protein A antibodies. The nitrocellulose sheets, treated as described above, with a test line as described above, were inserted into the resulting mixture, and the mixture migrated through the sheet, passed the test line (if applied) and the control line with the immobilized protein A, and into the absorption pad. Both the control line and the test line developed a red colour from the gold colloid particles.
In separate experiments, 20 ul of goat serum was added to the said mixtures, to illustrate result of leakage of plasma proteins into the urine sample (or presence of plasma proteins if the sample had been a blood sample). Presence of the plasma proteins, including the mammalian antibodies and the goat serum, did not interfere neither with the formation of the control line nor the test line.

The reason for this is that the irrelevant mammalian antibodies bind to Protein A with their constant chain (Fc chain) at a specific binding site on Protein A, while the antibodies raised by immunisation of the animal with Protein A binds on other epitopes/binding sites of Protein A (whether these are polyclonal or monoclonal of origin). Especially preferred are avian antibodies, since their Fc-chain does not bind to Protein A, and any kind of interfering reactions are thus avoided.

### Example 5 Synthesis of hemocyanin conjugated blue particles.

154 mg blue carboxylated polystyrene particles with a mean diameter of 185 nm from Merck Prolabo, France, was washed by centrifugation and re-suspension three times in 10 mM borate buffer with 15 mM sodium chloride. The particles were suspended to 5 ml in the same buffer solution.
40 mg Haemocyanin type VIII from Sigma, US, was dialysed against the said borate buffer solution.
6 mg EDC was dissolved in 1 ml of water at 4 degrees Celcius. Under stirring the EDC solution was added to the particles, and thereafter the hemocyanin solution was added. The suspension was thereafter stirred over night at room temperature, followed by washing by centrifugation and re-suspension in 25 ml 10 mM boratbuffer pH = 9.3 with 15 mM NaCl, 0.09 % NaAzide and 0.2 % Tween 20 added.
Thereafter the particles was washed by centrifugation and resuspension in 25 ml 10 mM boratbuffer pH = 8.7 with 15 mM NaCl, 0.09 % NaAzide and 0.2 % Tween 20 added.

### Example 6 Synthesis of hemocyanin conjugated green particles.

282 mg green carboxylated polystyrene particles with a mean diameter of 340 nm from Merck Prolabo, France, was washed by centrifugation and re-suspension three times in 10 mM borate buffer with 15 mM sodium chloride. The particles were suspended to 5 ml in the same buffer solution.
40 mg Hemocyanin type VIII from Sigma, US, was dialysed against the said borate buffer solution.
6 mg EDC was dissolved in 1 ml of water at 4 degrees Celcius. Under stirring the EDC solution was added to the particles, and thereafter the hemocyanin solution was added. The suspension was thereafter stirred over night at room temperature, followed by washing by centrifugation and re-suspension in 25 ml 10 mM boratbuffer pH = 9.3 with 15 mM NaCl, 0.09 % NaAzide and 0.2 % Tween 20 added.
Thereafter the particles was washed by centrifugation and re-suspension in 25 ml 10 mM boratbuffer pH = 8.7 with 15 mM NaCl, 0.09 % NaAzide and 0.2 % Tween 20 added.

### Example 7: Immobilising Hemocyanin on the control line.

HighFlow 120 nitrocellulose from Millipore, US, was cut in pieces approximately 4 mm x 70 mm, and an cellulose absorbance pad to absorb was attached to one end (hereafter named and B, the opposite end named end A).

To 10 mM ammonium acetate in water was added 2 vol. % ethanol. To this solution Hemocyanin type VIII from Sigma Inc., US, was dissolved 2 mg per ml. Using a syringe tip, this solution was applied across the nitrocellulose strip, 7 ul per cm, and left to dry for 2 hours at 37 degrees Celsius. On some of the sheets, a test line with anti- human chorion gonadotropin mouse monoclonal antibodies was applied using the same method, the test line being located a few mm upstream from the control line.
Thereafter, end A of the sheets were dipped into a blocking solution comprising 25 mM TRIS buffer, 150 mM NaCl, egg albumin 2 mg/ml, 100 ug/ml of irrelevant IgY, and this blocking solution migrated through the sheet and into the absorbance pad. Thereafter, the sheets were left to dry in 37 degrees Celsius.

Optionally, the blocking solution comprised 5 vol. % of goat blood serum, which gave an excellent blocking.

### Example 8: Immobilising anti-hemocyanin antibodies on the control line.

HighFlow 120 nitrocellulose from Millipore, US, was cut in pieces approximately 4 mm x 70 mm, and an cellulose absorbance pad to absorb was attached to one end (hereafter named and B, the opposite end named end A).

IgY from egg from hens having been immunised with Hemo cyanin type VIII in a suspension with Freund's complete adjuvance was isolated by conventional ammonium chloride precipitation technique (and optionally affinity purified by the use of Hemocyanin immobilised in a gel column, using conventional technique), all well described in the literature well known to the skilled man of the art. 30 mg of the antibodies were dialysed against 10 mM borate buffer 15 mM NaCl pH = 9.3 for 24 hours, and the volume was adjusted to 15 ml in the same buffer.

Using a syringe tip, this solution was applied across the nitrocellulose strip, 7 ul per cm, and left to dry for 2 hours at 37 degrees Celsius. On some of the sheets, a test line with anti- human chorion gonadotropin mouse monoclonal antibodies was applied using the same method, the test line being located a few mm upstream from the control line.

Thereafter, end A of the sheets were dipped into a blocking solution comprising 25 mM TRIS buffer, 150 mM NaCl, egg albumin 2 mg/ml, 100 ug/ml of irrelevant IgY, and this blocking solution migrated through the sheet and into the absorbance pad. Thereafter, the sheets were left to dry in 37 degrees Celsius.

Optionally, the blocking solution comprised 5 vol. % of goat blood serum, which gave an excellent blocking.

### Example 9: Demonstration of the use of anti- Hemocyanin antibodies on the control line and hemocyanin immobilised on green latex particles.

50 ul Urine samples, some comprising 1000 IU/l human chorion gonadotropic hormone (HCG), some without HCG, was mixed with 200 ul of a buffer comprising 25 mM TRIS buffer, 150 mM NaCl, egg albumin 2 mg/ml, 50 ug/ml of irrelevant IgY.
To create a control line, 25 ul of particle suspension comprising green latex with immobilized Hemocyanin (see above) was mixed with the urine/buffer mixture.

The nitrocellulose sheets, treated with anti-hemocyanin antibodies on the control line as described above, was inserted into the resulting mixture, and the mixture migrated through the sheet, passed the test line (if applied) and the control line with the immobilized anti- Hemocyanin antibodies , and into the absorption pad. A green control line developed. Coloured particles did not bind to the test line if present.
In separate experiments, 25 ul of gold colloid particles with immobilized anti-HCG antibodies was mixed to the said mixture comprising green latex particles with immobilized Hemocyanin. The nitrocellulose sheets, treated as described above, with a test line as described above, were inserted into the resulting mixture, and the mixture migrated through the sheet, passed the test line (if applied) and the control line with the immobilized anti-hemocyanin antibodies, and into the absorption pad. The control line developed a green colour and the test line developed a red colour.

### Example 10: Demonstration of the use of anti- Hemocyanin antibodies immobilised on blue latex particles and hemocyanin immobilised on the control line.

50 ul Urine samples, some comprising 1000 IU/l human chorion gonadotropic hormone (HCG), some without HCG, was mixed with 200 ul of a buffer comprising 25 mM TRIS buffer, 150 mM NaCl, egg albumin 2 mg/ml, 50 ug/ml of irrelevant IgY.
To create a control line, 25 ul of particle suspension comprising blue latex with immobilized anti-hemocyanin antibodies (see above) was mixed with the urine/buffer mixture.

The nitrocellulose sheets, treated with hemocyanin on the control line as described above, was inserted into the resulting mixture, and the mixture migrated through the sheet, passed the test line (if applied) and the control line with the immobilized Hemocyanin., and into the absorption pad. A blue control line developed. Coloured particles did not bind to the test line if present.
In separate experiments, 25 ul of gold colloid particles with immobilized anti-HCG antibodies was mixed to the said mixture comprising green latex particles with immobilized Hemocyanin. The nitrocellulose sheets, treated as described above, with a test line as described above, were inserted into the resulting mixture, and the mixture migrated through the sheet, passed the test line (if applied) and the control line with the immobilized hemocyanin, and into the absorption pad. The control line developed a blue colour and the test line developed a red colour.

## Claims

1. A control system for immunochromatographic systems, comprising a control reagent and a control detector being a binding pair constituted by an antigen and antibody towards said antigen, wherein said antigen origins from a species different from the species from which the analyte origins, and provided that the antigen is not an antibody per se, and wherein the antigen is Protein A or hemocyanin.

2. A control system according to claim 1, wherein the antigen of the said binding pair is immobilized on coloured microparticles.

3. A control system according to claim 1, wherein the antibody of the said binding pair is immobilized on coloured microparticles.

4. A control system according to claim 1, wherein the control reagent is protein A or Protein G, and wherein the control detector is an anti-Protein A-antibody or anti-Protein G-antibody, preferably an avian anti-Protein A-antibody or an avian anti-Protein G-antibody.

5. A control system according to claim 1, wherein the antigen is molluscan hemocyanin.

6. A control system according to claim 5, wherein said molluscan hemocyanin constitutes the control reagent and wherein the control detector is an anti-hemocyanin-antibody, preferably of avian origin.

7. A control system according to claim 5, wherein said molluscan hemocyanin constitutes the control detector and wherein the control reagent is an anti-hemocyanin-antibody, preferably of avian origin.

8. A control system according to any of the claims 1-7, wherein the control detector is labelled, preferably by immobilization on microparticles, preferably colloidal gold particles or coloured latex particles.

9. A control system according to any of the claims 1-7, wherein the analyte detector and the control detector is immobilized on microparticles, preferably latex particles of different colour.

10. A control line system, according to any of the claims 1-9, wherein Protein A is immobilized on the porous substrate in which the test is performed, either by physical adsorption or by chemical coupling.

11. A method for determining the efficacy of a chromatographic analysis, comprising the steps of providing a chromatographic analysis system, providing said system with a control system according to any of the claims 1-11, wherein the control systems comprises at least on control component being a antigen and exposing the test device to the test material, letting the test material migrate into the test device and inspecting the result of the test on the test area and the control area.

12. A method according to claim 12, wherein the chromatographic system comprises a chromatographic strip, wherein the control line is located adjacent to the test region.

13. A method according to claim 12, whereby anti-Protein A-antibody is labelled with coloured microparticles, preferably with microparticles, having a colour different from the colour of the test particles.

14. A method according to any of the claims 1-13, whereby the efficacy of the chromatographic system is determined by visualizing of the control reagent-control detector complex.

15. An immunochromatographic test device comprising a control system according to any of the claims 1-12.

16. An immunochromatographic test device according to claim 20, wherein the test device further comprises a chromatographic strip, and further wherein Protein A is immobilized on a control line and wherein the control detector is an anti-Protein A-antibody.

17. An immunochromatographic test device according to claim 20, wherein the test device further comprises a chromatographic strip, and further wherein hemocyanin is immobilized on a control line and wherein the control detector is an anti-hemocyanin-antibody.

18. An immunochromatographic test device according to claim 20, wherein the test device further comprises a chromatographic strip, and further wherein anti-hemocyanin-antibodies is immobilized on a control line and wherein the control detector is hemocyanin.

19. An immunochromatographic test device according to claims 19-22, wherein the anti-protein A-antibodies, the hemocyanin or the anti-hemocyanin-antibody is labelled, preferably by colloidal gold particles or blue latex particles.

20. An immunochromatographic test device according to claim 19, wherein the chromatographic strip is comprised by a porous and preferentially hydrophilic material, preferably comprised by nitrocellulose or another synthetic or a biologic derived filter material or a porous glass matrix, or combinations thereof.

21. An immunochromatographic test device according to any of the claims 15-20, wherein the control reagent is immobilized by physical adsorption and chemical coupling.

22. The use of a species-specific substance and an antibody derived against said substance as a control system in immunochromatographic methods and devices.

23. A control system for immunochromatographic systems, comprising a binding pair, where one of the members of the said binding pair is immobilized on a coloured microparticle, and wherein the colour of said coloured microparticle differs from the colour of the microparticle which in said immunochromatographic system is used to visualize the analyte to be detected by said immunochromatographic system, and wherein the members of said binding pair is protein A and anti-protein A antibody or hemocyanin and anti-hemocyanin antibody, respectively.
